## Europäisches Patentamt
## European Patent Office
## Office européen des brevets

(19)

(11) Numéro de publication: **O 033 987**
**B1**

(12) ## FASCICULE DE BREVET EUROPEEN

(45) Date de publication du fascicule du brevet: **13.04.83**

(51) Int. Cl.³: **C 12 M 1/26, C 12 N 1/16**

(21) Numéro de dépôt: **81200053.7**

(22) Date de dépôt: **19.01.81**

(54) Perfectionnements aux procédés et installations utilisant des levures.

(30) Priorité: **07.02.80 LU 82145**

(43) Date de publication de la demande:
**19.08.81 Bulletin 81/33**

(45) Mention de la délivrance du brevet:
**13.04.83 Bulletin 83/15**

(84) Etats contractants désignés:
**AT BE CH DE FR GB IT LI NL SE**

(56) Documents cités:
**BE - A - 670 985**
**CH - A - 386 362**
**FR - A - 2 228 407**
**FR - A - 2 259 903**
**GB - A - 1 103 757**

(73) Titulaire: **COMPAGNIE INTERNATIONALE DE PARTICIPATION ET D'INVESTISSEMENT CIPARI S.A.**
**4-10 Boulevard d'Avranches**
**Luxembourg (LU)**

(72) Inventeur: **Devreux, André Fernand Oscar**
**60 Rue Buisseret**
**B-7000 Mons (BE)**

(74) Mandataire: **De Brabanter, Maurice et al,**
**Bureau VANDER HAEGHEN 63 Avenue de la Toison d'Or**
**B-1060 Bruxelles (BE)**

Courier Press, Leamington Spa, England.

Perfectionnements aux procédés et installations utilisant des levures.

La présente invention est relative à des perfectionnements aux procédés et installations utilisant des levures pour la fabrication de boissons, telles que bières, cidres, vins et autres boissons alcoolisées, ainsi que de produits de boulangerie.

Il est connu, en brasserie, de stocker une suspension aqueuse de levure ou levain provenant d'une culture de levure produite dans un propagateur et/ou de la récolte de levure précédemment utilisée dans la fabrication du moût, dans un réservoir de stockage dénommé "levurier", d'où une quantité déterminée de suspension de levure est soutirée et envoyée par des moyens, tels qu'une pompe doseuse, dans une cuve de fermentation de moût. La pompe doseuse est insérée dans une conduite reliant le levurier à la cuve de fermentation.

On sait, par ailleurs, que la qualité de la suspension de levure contenue dans le levurier est variable, de sorte que la quantité de cette suspension à introduire dans la cuve de fermentation est, elle aussi, variable selon divers facteurs, tels que la compacité de la levure, son pouvoir fermentaire, etc.

Diverses méthodes sont utilisées, de manière connue, pour régler la quantité volumétrique et pondérale de suspension de levure à utiliser dans une cuve de fermentation de capacité donnée. Une de ces méthodes consiste à prélever un échantillon de la levure dans le levurier et de mesurer, en laboratoire, la compacité de cette levure par centrifugation. Une autre méthode consiste à injecter une quantité minimale de suspension de levure dans la cuve de fermentation, puis d'attendre que cette levure soit bien répartie dans le moût, après quoi on prélève un échantillon de moût fermenté contenant de la levure, on procède à un comptage du nombre de cellules de levure contenu dans cet échantillon et, en fonction des résultats de ce comptage, on introduit une quantité supplémentaire de levure dans la cuve de fermentation pour assurer une fermentation optimale de moût dans cette cuve.

Ces méthodes connues présentent divers inconvénients, notamment celui de ne pas permettre une fermentation qualitativement uniforme du moût. La première méthode connue évoquée dans le paragraphe précédent a, de plus, l'inconvénient de prendre beaucoup de temps, le prélèvement d'un échantillon et la mesure de la compacité de la suspension de levure en laboratoire prenant beaucoup de temps, de sorte qu'il n'est pratiquement pas possible de déterminer, en temps utile, le pouvoir fermentaire de la levure utilisée. Quant au second procédé connu qui implique un comptage des cellules de levure, il manque de précision, du fait que ce comptage ne tient pas compte des cellules mortes ou des cellules dont le pouvoir fermentaire a été altéré.

La présente invention vise à remédier à ces inconvénients.

Elle a tout d'abord pour object un procédé de réglage de la quantité de levure à introduire dans une enceinte de fermentation contenant un liquide fermentescible.

Le procédé suivant l'invention se caractérise essentiellement par le fait qu'on prélève, à des moments prédéterminés, un échantillon de volume déterminé d'une masse de levure en suspension pendant que cette masse est maintenue en mouvement dans un circuit fermé, on soumet cet échantillon à une fermentation en y ajoutant une quantité prédéterminée d'un élément nutritif et on règle l'alimentation en levure de l'enceinte de fermentation en fonction du pouvoir fermentaire de l'échantillon de levure traité.

Selon une particularité du procédé suivant l'invention, on règle l'alimentation en levure de l'enceinte de fermentation en fonction, par exemple, de l'augmentation maximale de pression provoquée par la fermentation de l'échantillon de levure prélevé, l'échantillon de levure étant, de préférence, prélevé en aval des moyens, tels que la pompe doseuse, destinés à alimenter l'enceinte de fermentation contenant le liquide fermentescible.

L'invention concerne aussi une installation de réglage de la quantité de levure à introduire dans une enceinte de fermentation, comprenant un réservoir à fond conique contenant de la levure en suspension dans un liquide et une conduite reliant le fond de ce réservoir à au moins une cuve de fermentation, ainsi que des moyens, tels qu'une pompe doseuse permettant l'alimentation de la cuve de fermentation en levure à partir du réservoir de levure.

L'installation suivant l'invention se caractérise essentiellement par le fait qu'elle comporte une conduite de recyclage de la levure en suspension contenue dans le réservoir susdit, ainsi que des moyens pour assurer, pendant un temps prédéterminé, le recyclage de la levure en suspension de la partie inférieure du réservoir susdit à la partie supérieure de celui-ci, des moyens pour prélever sur la conduite de recyclage susdite, à des moments choisis, un échantillon de volume prédéterminé de la suspension de levure en cours de recyclage et pour envoyer cet échantillon dans un petit fermenteur alimenté en élément nutritif, des moyens pour déterminer le pouvoir fermentaire de l'échantillon de levure dans le fermenteur et des moyens pour régler l'alimentation en levure de la cuve de fermentation en fonction du pouvoir fermentaire de l'échantillon de levure, déterminé dans le fermenteur.

Suivant une particularité de l'installation suivant l'invention, une pompe doseuse d'alimentation en levure de la cuve de fermentation est montés sur la conduite reliant le fond du réservoir de levure à la cuve de fermentation, en amont de la conduite de recyclage de la levure dans le réservoir précité. Les moyens de prélèvement d'échantillons de levure sont avantageusement constitués par une vanne qui, dans une position, envoie ou permet l'envoi d'une quantité prédéterminée de levure en suspension dans le fermenteur. La vanne susdite peut, dans le cadre de l'invention, être une vanne à plus de deux voies qui, dans une première position, permet le recyclage de la levure en suspension dans le réservoir de levure, dans une seconde position, permet le prélèvement d'un échantillon de levure et l'envoi de cet échantillon dans le fermenteur, et dans une troisième position, assure une liaison entre une source d'un liquide de lavage et le fermenteur ou entre une source d'air comprimé et le fermenteur.

L'installation suivant l'invention comporte aussi, selon une de ses caractéristiques, des moyens pour régler la durée de marche de la pompe doseuse susdite en fonction du pouvoir fermentaire de l'échantillon de levure déterminé dans le fermenteur.

D'autres particularités et détails de l'invention ressortiront de la description détaillée suivante des dessins ci-annexés qui montrent schématiquement et à seul titre illustratif une forme de réalisation d'une installation suivant l'invention.

Dans ces dessins:

— la figure 1 est une coupe verticale d'une installation suivant l'invention;

— la figure 2 est une coupe d'une partie de l'installation suivant l'invention, montrant comment se fait le prélèvement d'échantillons de levure dans le circuit de recyclage de la levure contenue dans le réservoir de stockage de celle-ci;

— la figure 3 est une coupe verticale semblable à celle de la figure 2 suivant l'invention, montrant comment se fait le nettoyage de l'installation.

Dans ces différentes figures, les mêmes notations de référence désignent des éléments identiques.

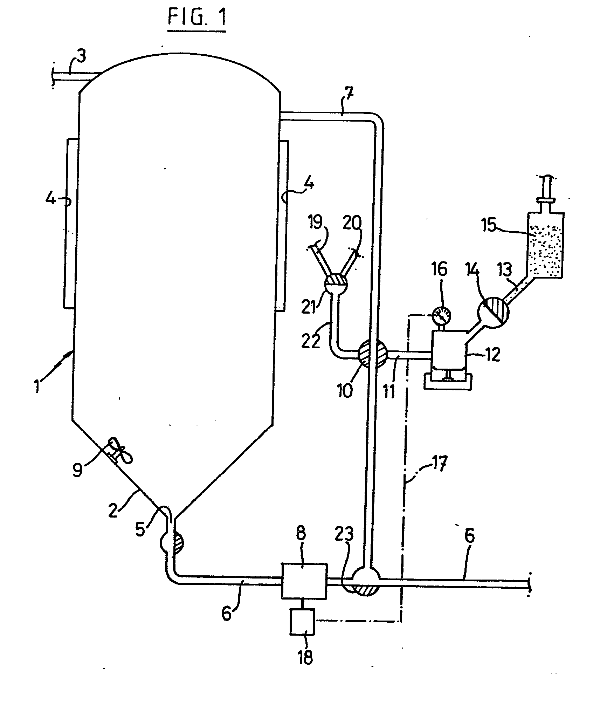

La figure 1 montre un réservoir 1 de stockage d'une suspension de levure dans un liquide, tel que du moût de brasserie. Ce réservoir est, de préférence, un réservoir cylindrique à fond conique 2. Il est destiné à recevoir par une conduite 3 une suspension de levure venant d'un dispositif de culture de levure fraîche et/ou d'un cuve de fermentation de moût de bière (non montrée). Le réservoir de stockage de levure 1 est entouré d'une chemise de réfrigération 4 dans laquelle circule un agent réfrigérant destiné à maintenir la suspension de levure à une température d'environ 0°C dans ledit réservoir 1.

A l'extrémité inférieure 5 du réservoir 1 de stockage de levure ou levurier est reliée une conduite d'alimentation 6 servant à amener la suspension de levure du réservoir de stockage 1 à la cuve de fermentation de moût de bière.

Sur la conduite d'alimentation est branchée une conduite de recyclage 7 qui aboutit à la partie supérieure du réservoir 1. Cette conduite de recyclage 7 est, de préférence, montée en aval des moyens, tels que d'une pompe doseuse 8 qui sert à véhiculer la suspension de levure à partir du levurier 1 vers la cuve de fermentation. Cependant, la conduite de recyclage 7 peut être branchée sur la conduite d'alimentation 6 en amont de la pompe doseuse 8, auquel cas une pompe de recyclage particulière doit être prévue dans le circuit de recyclage de la suspension de levure, ce circuit étant constitué par la conduite de recyclage 7 et le levurier 1 qui est équipé, de préférence au voisinage de son fond conique, d'un agitateur 9, par exemple du type électromagnétique connu en soi.

Dans la conduite de recyclage 7 est montée une vanne à quatre voies 10 qui, dans la position montrée à la figure 1, permet un recyclage continu de levure en suspension de l'extrémité inférieure du levurier 1 à sa partie supérieure. Grâce à l'agitateur 9 et à la conduite de recyclage, il est possible de maintenir une composition homogène de la suspension de levure dans le réservoir de stockage 1, de sorte qu'au moment où l'on désire prélever un échantillon de levure, comme décrit plus loin, cet échantillon est homogène et est réellement représentatif de la qualité moyenne de la suspension de levure stockée dans le réservoir 1.

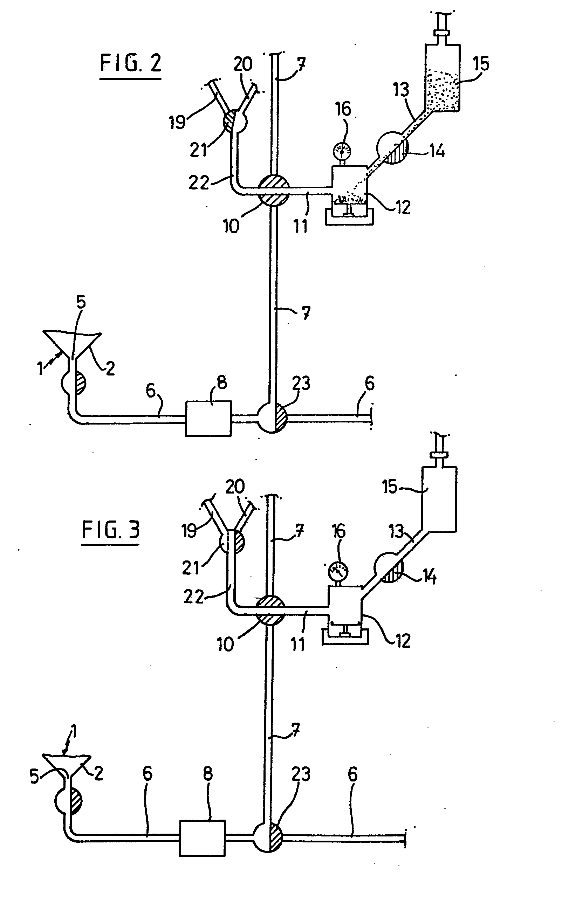

A la vanne 10 est reliée une conduite 11 de prélèvement d'échantillons qui aboutit à un fermenteur 12 dans lequel est recueilli un échantillon de levure en suspension, de poids ou de volume prédéterminé, lorsque la vanne 10 a été amenée dans la position montrée à la figure 2. L'échantillon recueilli dans le fermenteur 12 est additionné d'un liquide nutritif venant, par une conduite 13 munie d'un robinet 14, d'un récipient 15 contenant une réserve d'un liquide nutritif, tel qu'une solution aqueuse de sucre. La quantité de liquide nutritif ajoutée à l'échantillon de levure recueilli dans le fermenteur 12 est réglée par le temps ou le degré d'ouverture du robinet 14.

Dans le fermenteur 12, l'échantillon de levure est porté par un dispositif approprié de chaffage à une température adéquate pour provoquer une fermentation ou croissance de la levure entraînant un dégagement d'anhydride carbonique. Au fermenteur 12 est associé un dispositif 16 qui peut être un appareil de détection de pression, de pH ou d'autres modifications ou phénomènes, qui traduit la capacité de fermentation de l'échantillon de levure et qui commande, comme montré schématiquement par la ligne en traits interrompus 17, le moteur 18 de commande de la pompe doseuse 8, en sorte que la durée de fonctionnement de cette pompe ou son débit et, par conséquent, la quantité de levure en suspension envoyée du levurier 1 par la conduite d'alimentation 6 à la cuve de fermentation, sont réglés en fonction de la qualité de la levure dans le levurier 1. Le pouvoir de fermentation de cette levure peut être mesuré à tout moment, par prélèvement d'un échantillon dans le fermenteur 12, dans lequel la qualité de la levure peut être testée en quelques minutes.

Lors de l'interruption de la marche de l'installation, celle-ci peut être nettoyée en maintenant la vanne 10 dans la position illustrée à la figure 2 et en permettant d'abord l'admission d'un liquide de nettoyage, tel qu'une solution de soude caustique venant d'un réservoir (non montré) par une conduite 19, puis l'admission d'aire comprimé par une conduite 20, les conduites 19 et 20 étant reliées à une vanne à trois voies 21 connectée à une conduite 22 raccordée à la vanne précitée 10.

Une vanne 23 ou un dispositif analogue peut être utilisé pour mettre l'installation suivant l'invention hors service ou en service.

Le prélèvement d'un échantillon de levure peut, dans le cadre de la présente invention, s'effectuer pendant que la pompe doseuse alimente la cuve de fermentation en levure, auquel cas le dispositif de mesure du pouvoir de fermentation associé au fermenteur 12 commande l'arrêt de la pompe doseuse ou son débit de telle façon qu'une quantité adéquate de levure soit envoyée dans la cuve de fermentation, en fonction de la qualité de la levure.

Il est évident que l'invention n'est pas limitée aux détails décrits plus haut et que de nombreuses modifications peuvent être apportées à ces détails sans sortir du cadre de l'invention.

Ainsi, bien que le procédé et l'installation suivant l'invention aient été décrits plus haut dans leur application en brasserie, l'homme de métier pourra aisément adapter ledit procédé et ladite installation aux domaines de la fabrication de cidre, de vin, voire d'autres boissons alcoolisées. Le procédé de l'invention est applicable, de manière générale, à tous les domaines industriels où il est fait usage de levure, notamment en boulangerie.

**Revendications**

1. Procédé de réglage de la quantité de levure à introduire dans une enceinte de fermentation contenant un liquide fermentescible, caractérisé en ce qu'on prélève, à des moments prédéterminés, un échantillon de volume déterminé d'une masse de levure en suspension pendant que cette masse est maintenue en mouvement dans un circuit fermé, on soumet cet échantillon à une fermentation en y ajoutant une quantité prédéterminée d'un élément nutritif et on règle l'alimentation en levure de l'enceinte de fermentation en fonction du pouvoir fermentaire de l'échantillon de levure traité.

2. Procédé suivant la revendication 1, caractérisé en ce qu'on soumet au surplus la masse de levure en suspension à une agitation.

3. Procédé suivant l'une ou l'autre des revendications précédentes, caractérisé en ce qu'on règle l'alimentation en levure de l'enceinte de fermentation en fonction d'une modification ou d'un phénomène provoqué par la fermentation de l'échantillon de levure prélevé.

4. Procédé suivant l'une ou l'autre des revendications précédentes, caractérisé en ce qu'on prélève l'échantillon de levure en aval de moyens, tels qu'une pompe doseuse, destinés à alimenter l'enceinte de fermentation contenant le liquide fermentescible.

5. Procédé suivant l'une ou l'autre des revendications précédentes, caractérisé en ce que le liquide de fermentation est du moût de brasserie.

6. Installation de réglage de la quantité de levure à introduire dans une enceinte de fermentation, comprenant un réservoir à fond conique contenant de la levure en suspension dans un liquide et une conduite reliant le fond de ce réservoir à au moins une cuve de fermentation, ainsi qu'une pompe doseuse permettant l'alimentation de la cuve de fermentation en levure à partir du réservoir de levure, caractérisée en ce qu'elle comporte une conduite de recyclage de la levure en suspension contenue dans le réservoir susdit, ainsi que des moyens pour assurer, pendant un temps prédéterminé, le recyclage de la levure en suspension de la partie inférieure du réservoir susdit à la partie supérieure de celui-ci, des moyens pour prélever sur la conduite de recyclage susdite, à des moments choisis, un échantillon de volume prédéterminé de la suspension de levure en cours de recyclage, et pour envoyer cet échantillon dans un petit fermenteur alimenté en élément nutritif, des moyens pour déterminer le pouvoir fermentaire de l'échantillon de levure dans le fermenteur et des moyens pour régler l'alimentation en levure de la cuve de fermentation en fonction du pouvoir fermentaire de l'échantillon de levure, déterminé dans le fermenteur.

7. Installation suivant la revendication 6, caractérisée en ce qu'une pompe doseuse d'alimentation en levure de la cuve de fermentation est montée sur la conduite reliant le fond du réservoir de levure à la cuve de fermentation, en amont de la conduite de recyclage de la levure dans le réservoir précité.

8. Installation suivant l'une ou l'autre des revendications 6 et 7, caractérisée en ce que le réservoir de levure est muni d'un agitateur.

9. Installation suivant l'une ou l'autre des revendications 6 à 8, caractérisée en ce que les moyens de prélèvement d'échantillons de levure sont constitués par une vanne qui, dans une position, permet l'envoi d'une quantité prédéterminée de levure en suspension dans le fermenteur.

10. Installation suivant la revendication 9, caractérisée en ce que la vanne susdite est une vanne à plus de deux voies qui, dans une première position, permet le recyclage de la levure en suspension dans le réservoir de levure, dans une seconde position, permet le prélèvement d'un échantillon de levure et l'envoi de cet échantillon dans le fermenteur, et dans une troisième position, assure une liaison entre une source d'un liquide de lavage et le fermenteur ou entre une source d'air comprimé et le fermenteur.

11. Installation suivant l'une ou l'autre des revendications 6 à 10, caractérisée en ce qu'elle comporte des moyens pour régler la durée de marche ou le débit de la pompe doseuse susdite en fonction du pouvoir fermentaire de l'échantillon de levure, déterminé dans le fermenteur.

12. Installation suivant l'une ou l'autre des revendications 6 à 11, caractérisée en ce que le fermenteur est muni d'un dispositif déterminant une modification ou un phénomène intervenant dans le fermenteur, le fonctionnement de la pompe doseuse étant asservi à ce dispositif.

13. Installation suivant l'une ou l'autre des revendications 6 à 12, caractérisée en ce qu'un récipient contenant un liquide nutritif est relié par une conduite d'alimentation au fermenteur, une vanne permettant de régler la quantité de liquide nutritif introduit dans le fermenteur en fonction de la quantité de l'échantillon de levure prélevé et envoyé dans le fermenteur.

14. Installation suivant l'une ou l'autre des revendications 6 à 13, caractérisée en ce que la conduite de recyclage est munie d'une vanne permettant d'interrompre la circulation de levure en suspension dans cette conduite de recyclage.

**Patentansprüche**

1. Verfahren zur Regelung der in einen eine Gärungsflüssigkeit enthaltenden Gärraum einzubringenden Hefemenge, dadurch gekennzeichnet, daß zu vorbestimmten Zeitpunkten eine Probe von bestimmtem Volumen einer in Suspension befindlichen Hefemasse entnommen wird, während diese Masse in einem geschlossenen Kreis in Bewegung gehalten wird, diese Probe durch Zugabe einer vorbestimmten Menge eines Nährstoffs einer Gärung unterzogen wird, und die Speisung des Gärraumes mit Hefe in Abhängigkeit von der Gärfähigkeit de behandelten Hefeprobe geregelt wird.

2. Verfahren nach Anspruch 1, dadurch gekennzeichnet, daß außerdem die in Suspension befindliche Hefemasse einer Rührung unterworfen wird.

3. Verfahren nach dem einen oder anderen der vorhergehenden Ansprüche, dadurch gekennzeichnet, daß die Speisung des Gärraumes mit Hefe in Abhängigkeit einer Veränderung oder eines durch die Gärung der entnommenen Hefeprobe hervorgerufenen Phänomens geregelt wird.

4. Verfahren nach dem einen oder anderen der vorhergehenden Ansprüche, dadurch gekennzeichnet, daß die Hefeprobe stromabwärts von zur Speisung des die Gärungsflüssigkeit enthaltenden Gärraumes bestimmten Einrichtungen, wie einer Dosierpumpe, entnommen wird.

5. Verfahren nach dem einen oder anderen der vorhergehenden Ansprüche, dadurch gekennzeichnet, daß die Gärungsflüssigkeit Brauereiwürze ist.

6. Vorrichtung zur Regelung der in einen Gärraum einzubringenden Hefemenge, mit einem Behälter mit konischem Boden zur Aufnahme der in einer Flüssigkeit in Suspension befindlichen Hefe und einer den Boden dieses Behälters mit zumindest einem Gärbottich verbindenden Leitung sowie einer die Speisung des Gärbottichs mit Hefe aus einem Hefebehälter zulassenden Dosierpumpe, dadurch gekennzeichnet, daß sie eine Rückflußleitung für die im Behälter enthaltene Hefesuspension aufweist, sowie Einrichtungen zur Gewährleistung des Rückflusses der Hefesuspension vom unteren Teil des Behälters zum oberen Teil desselben während eines bestimmten Zeitraumes, Einrichtungen zur Entnahme einer Probe vorbestimmten Volumens der im Rückfluß befindlichen Hefesuspension an der Rückflußleitung zu gewählten Zeitpunkten und zum Transport dieser Probe in einen mit Nährstoff gespeisten kleinen Fermenter, Einrichtungen zur Bestimmung der Gärfähigkeit der Hefeprobe im Fermenter und Einrichtungen zur Regelung der Speisung des Gärbottichs mit Hefe in Abhängigkeit von der im Fermenter bestimmten Gärfähigkeit der Hefeprobe.

7. Vorrichtung nach Anspruch 6, dadurch gekennzeichnet, daß eine Dosierpumpe zur Speisung des Gärbottichs mit Hefe an der den Boden des Hefebehälters mit dem Gärbottich verbindenden Leitung stromaufwärts der Rückflußleitung der Hefe in den genannten Behälter montiert ist.

8. Vorrichtung nach dem einen oder anderen der Ansprüche 6 und 7, dadurch gekennzeichnet, daß der Hefebehälter mit einem Rührer versehen ist.

9. Vorrichtung nach dem einen oder anderen der Ansprüche 6 bis 8, dadurch gekennzeichnet, daß die Einrichtungen zur Entnahme von Hefeproben durch ein Ventil gebildet sind, welches in einer Stellung die Förderung einer vorbestimmten Menge an Hefesuspension in den Fermenter gestattet.

10. Vorrichtung nach Anspruch 9, dadurch gekennzeichnet, daß das Ventil ein Mehrwegventil ist, welches in einer ersten Stellung den Rückfluß der Hefesuspension in den Hefebehälter gestattet, in einer zweiten Stellung die Entnahme einer Hefeprobe und die Förderung dieser Probe in den Fermenter gestattet und in einer dritten Stellung eine Verbindung zwischen einer Waschflüssigkeitsquelle und dem Fermenter oder zwischen einer Druckluftquelle und dem Fermenter herstellt.

11. Vorrichtung nach dem einen oder anderen der Ansprüche 6 bis 10, dadurch gekennzeichnet, daß sie Einrichtungen zur Regelung der Laufzeit oder der Durchflußmenge der Dosierpumpe in Abhängigkeit von der im Fermenter bestimmten Gärfähigkeit der Hefeprobe umfaßt.

12. Vorrichtung nach dem einen oder anderen der Ansprüche 6 bis 11, dadurch gekennzeichnet, daß der Fermenter mit einer eine im Fermenter auftretende Veränderung oder Phänomen bestimmenden Einrichtung versehen ist, wobei das Funktionieren der Dosierpumpe von dieser Einrichtung abhängig ist.

13. Vorrichtung nach dem einen oder anderen der Ansprüche 6 bis 12, dadurch gekennzeichnet, daß ein eine Nährflüssigkeit enthaltender Behälter über eine Zuführungsleitung mit dem Fermenter verbunden ist, wobei ein Ventil die Regelung der in den Fermenter eingebrachten Nährflüssigkeitsmenge in Abhängigkeit von der Menge der entnommenen und in den Fermenter geförderten Hefeprobe gestattet.

14. Vorrichtung nach dem einen oder anderen der Ansprüche 6 bis 13, dadurch gekennzeichnet, daß die Rückflußleitung mit einem Ventil versehen ist, welches die Unterbrechung der Zirkulation der Hefesuspension in dieser Rückflußleitung gestattet.

**Claims**

1. Method of controlling the quantity of yeast to be introduced into a fermentation vessel containing a fermentable liquid, characterised in that, at predetermined instants, one takes a sample of determined volume of a mass of yeast in suspension while that mass is maintained in movement in a closed circuit, one subjects that sample to fermentation while adding to it a predetermined quantity of a nutritive element and one controls the feed of yeast to the fermentation vessel as a function of the fermenting power of the sample of yeast treated.

2. Method according to claim 1, characterised in that furthermore one subjects the mass of yeast in suspension to agitation.

3. Method according to one or other of the preceding claims, characterised in that one controls the feed of yeast to the fermentation vessel as a function of a modification or of a phenomenon promoted by the fermentation of the sample of yeast taken.

4. Method according to one or other of the preceding claims, characterised in that one takes the sample of yeast downstream of means, such as a dosing pump, intended to feed the fermentation vessel containing the fermentable liquid.

5. Method according to one or other of the preceding claims, characterised in that the fermenting liquid is a brewery nutrient liquid.

6. Installation for control of the quantity of yeast to be introduced into a fermentation vessel, comprising a reservoir with a conical bottom containing yeast in suspension in a liquid and a conduit connecting the bottom of this reservoir to at least one fermentation vat, as well as a dosing pump permitting feeding of the fermentation vat with yeast from the yeast reservoir, characterised in that it includes a conduit for recirculation of yeast in suspension contained in the said reservoir, as well as means for performing, during a predetermined period, the recirculation of the yeast in suspension from the lower part of the said reservoir to the upper part of the latter, means for taking from the said recirculation conduit, at chosen instants, a sample of predetermined volume of the suspension of yeast in the course of recirculation, and for conveying that sample into a small fermenter fed with a nutrient element, means for determining the fermenting power of the sample of yeast in the fermenter, and means for controlling the feed of yeast to the fermentation vat as a function of the permenting power of the sample of yeast, determined in the fermenter.

7. Installation according to claim 6, characterised in that a dosing pump for feed of yeast to the fermentation vat is mounted on the conduit connecting the bottom of the reservoir for yeast to the fermentation vat, upstream of the conduit for recirculation of the yeast into the said reservoir.

8. Installation according to one or other of claims 6 and 7, characterised in that the reservoir for yeast is provided with an agitator.

9. Installation according to one or other of claims 6 to 8, characterised in that the means for taking samples of yeast are constituted by a valve member which, in one position, permits the conveyance of a predetermined quantity of yeast in suspension into the fermenter.

10. Installation according to claim 9, characterised in that the said valve member is a valve member with more than two ways which, in a first position, permits the recirculation of the yeast in suspension into the reservoir of yeast, in a second position permits the taking of a sample of yeast and transmitting that sample into the fermenter, and in a third position provides a connection between a source of a washing liquid and the fermenter or between a source of compressed air and the fermenter.

11. Installation according to one or other of claims 6 to 10, characterised in that it includes means for controlling the period of operation or discharge of said dosing pump as a function of the fermenting power of the sample of yeast, determined in the fermenter.

12. Installation according to one or other of claims 6 to 11, characterised in that the fermenter is provided with a device detecting a modification or a phenomenon taking place in the fermenter, the functioning of the dosing pump being dependent on this device.

13. Installation according to one or other of claims 6 to 12, characterised in that a receiver containing a nutrient liquid is connected by a feed conduit to the fermenter, a valve member permitting control of the quantity of nutrient liquid introduced into the fermenter as a function of the quantity of the sample of yeast taken and conveyed into the fermenter.

14. Installation according to one or other of claims 6 to 13, characterised in that the recirculation conduit is provided with a valve member permitting interruption of the circulation of yeast in suspension in this recirculation conduit.

**0 033 987**

FIG. 1

FIG. 2

FIG. 3